## Europäisches Patentamt
## European Patent Office
## Office européen des brevets

(19)

(11) Veröffentlichungsnummer: **0 045 041**
**B1**

(12) **EUROPÄISCHE PATENTSCHRIFT**

(45) Veröffentlichungstag der Patentschrift:
**09.05.84**

(51) Int. Cl.³: **C 12 M 1/20**

(21) Anmeldenummer: **81105727.2**

(22) Anmeldetag: **21.07.81**

(54) **Nährbodenträgersystem.**

(30) Priorität: **25.07.80 DE 3028204**

(43) Veröffentlichungstag der Anmeldung:
**03.02.82 Patentblatt 82/5**

(45) Bekanntmachung des Hinweises auf die Patenterteilung:
**09.05.84 Patentblatt 84/19**

(84) Benannte Vertragsstaaten:
**AT BE CH DE FR GB IT LI NL SE**

(56) Entgegenhaltungen:
**FR - A - 1 545 451**
**FR - A - 2 213 981**

(73) Patentinhaber: **Biotest-Serum-Institut GmbH,
Flughafenstrasse 4, D-6000 Frankfurt-Niederrad (DE)**

(72) Erfinder: **Fiebig, Reinhard, In der Eisenbach 35,
D-6270 Idstein/Taunus (DE)**
Erfinder: **Schleussner, Hans, Dr. Dipl.-Chem.,
Nansenring 26, D-6000 Frankfurt (DE)**

(74) Vertreter: **Beil, Walter, Dr. et al, BEIL, WOLFF & BEIL
Rechtsanwälte Adelonstrasse 58, D-6230 Frankfurt am
Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Nährbodenträgersystem aus Nährbodenträger, Umhüllung und einer Siegelfolie.

Mit Nährboden gefüllte Nährbodenträger dienen dem Zweck der Züchtung von Keimen und der Keimbestimmung.

Zur Beimpfung mit Keimen verwendete man bisher sogenannte Petrischalen, deren Boden mit einem Nährboden beschichtet ist, und die entweder rund oder viereckig sein können und deren Boden glatt oder unterteilt sein kann. Diese Schalen werden mit einem darüberzustülpenden Deckel verschlossen (vgl. Greiner Labortechnik für Medizin und Forschung (1976), S. 35-36).

Zur Erkennung lokalisierter Keime dienen Abklatschkulturen oder sogenannte Dip-slides. Für Abklatschkulturen verwendete man bisher Kontakt-Schalen, die ähnlich wie Petrischalen gestaltet sind, jedoch einen niedrigen Rand besitzen, über den der Nährboden zum Zwecke des Abklatschens herausragt (vgl. a.a.o.S. 37).

Die sogenannten Dip-slides oder Tauchstreifen sind mit einem Nährmedium beschichtete Streifen, die in einem Röhrchen mittels eines Stopfens oder ähnlichen Verschlusses keimdicht verschlossen sind und die zur Keimbestimmung in flüssigen Medien verwendet werden können.

Aus der DE-C 2301385 ist ausserdem eine Vorrichtung zur Prüfung der Luft auf ihren Keimgehalt bekannt, bei der eine mit Nährboden beschichtete Trägerfolie, die in ihrer Länge dem Umfang der Vorrichtung entspricht, und die in Näpfchen zur Aufnahme des Nährbodens aufgeteilt ist, verwendet wird.

Sowohl Petrischalen als auch Kontaktschalen und Dip-slides sind relativ teuer, wobei die beiden Schalenarten ausserdem ein hohes Risiko der Verunreinigung, insbesondere auch beim Versand, in sich bergen, da sie vor dem Gebrauch nicht fest versiegelt sind.

Darüber hinaus erfordert jede der drei Behältnisarten eine gesonderte Fertigung, gesonderte Fertigungswerzeuge etc. und der Verbraucher, der sich mit allen drei Keimbestimmungsarten befasst, benötigt für jedes dieser Behältnisse eine besondere Lagerhaltung.

Petrischalen werden praktisch seit Robert Kochs Zeiten, d.h. seit etwa 100 Jahren im wesentlichen unverändert für mikrobiologische Versuche verwendet, wobei man heute lediglich neben dem ursprünglich verwendeten Glas auch Kunststoff als Schalenmaterial einsetzt. Das System der Petrischale konnte solange als einigermassen befriedigend und brauchbar angesehen werden, solange es vorwiegend für den Eigenbedarf eines kleinen Verbraucherkreises hergestellt und die Versuche im kleinen Rahmen durchgeführt wurden.

Mit zunehmender Technisierung und Rationalisierung erwuchs der Bedarf an einem System, das leichter und preiswerter herstellbar ist, standardisierte Qualität aufweist und vor Sekundärkontamination geschützt ist.

Die in der DE-C 2301385 beschriebene Folie weist zwar auch eine keimdichte Verpackung auf, jedoch lässt sich nach Entfernen der Siegelfolie und nach Verwendung zur Prüfung der Luft auf ihren Keimgehalt der Nährbodenträger nicht mehr selbsttätig an der Umhüllung fixieren. Das System kann nur mit Hilfe eines Klebestreifens oder eines Schiebers oder ähnlicher Massnahmen verschlossen werden. Ausserdem ist die Folie so gestaltet, dass sie sich speziell nur in der für sie gedachten Vorrichtung verwenden lässt.

Aufgabe der Erfindung war es somit, ein hermetisch verschlossenes keimdichtes Mehrzweck-Behältnis, das sowohl die Petrischale ersetzen soll, als auch für Abklatschkulturen und Keimbestimmung in Flüssigkeiten verwendbar ist, bereitzustellen, das sich auf rationelle Weise kostengünstig fertigen lässt, dessen keimdichter Verschluss auch während des Versandes gewährleistet ist und das sich nach Beimpfung bis zur Bebrütung wieder ausreichend verschliessen lässt.

Diese Aufgabe wird erfindungsgemäss durch ein Nährbodenträgersystem aus Nährbodenträger 1, Umhüllung 2 und Siegelfolie 3 gelöst, das dadurch gekennzeichnet ist, dass der Nährbodenträger 1 eine zur Aufnahme des Nährbodens bestimmte Vertiefung 4, die durch Stege 5 in Felder 6 unterteilt ist, einen von der Nährbodenfläche nach oben gezogenen umlaufenden Wulstrand 7, an einem Ende ein Griffstück 8 und einen um beide Seiten und das andere Ende herumlaufenden flachen Rand 9 aufweist und die Umhüllung 2 eine Vertiefung 10 und einen umlaufenden flachen Rand 11 zur Aufnahme der Siegelfolie 3 aufweist, wobei die Vertiefung 10 an zwei Seiten und einem Ende sich zum flachen Rand 11 hin stufenförmig auf die Ausmasse des Nährbodenträgers 1 verbreitert, der engere Teil so bemessen ist, dass die beiden Seitenwände und ein Ende den Wulstrand 7 an zwei Seiten und an dem dem Griffstück 8 gegenüberliegenden Ende berühren, der Stufenabsatz 12 an seinem oberen Rand Festhaltestege 13 aufweist, die den Nährbodenträger 1, dessen flacher Rand 9 auf der Stufe aufliegt, festhalten, und die Vertiefung 10 an der Seite, an der das Griffstück 8 des Nährbodenträgers 1 zu liegen kommt, abgerundet oder abgeschrägt ist und die Rundung oder Schräge mit einer Aussparung 14 zum Greifen und Herausziehen des Nährbodenträgers 1 versehen ist und wobei Nährbodenträger 1 und Umhüllung 2 aus sterilisierbarer Kunststoff-Folie bestehen.

Der Nährbodenträger, die Umhüllung und die Siegelfolie bestehen aus einer sterilisierbaren Folie, wobei sich die beiden ersten auf fertigungstechnisch einfache Weise mit Hilfe einer Tiefzieheinrichtung herstellen lassen. Als sterilisierbarer Kunststoff wird vorzugsweise thermoplastisches Material verwendet, wie beispielsweise PVC, Polyester, Polystyrol, Polyethylen und Polypropylen.

Der Nährbodenträger wird mit Nährboden beschichtet, mit der Schicht nach unten in die Umhüllung eingelegt und mit der Siegelfolie dadurch hermetisch verschlossen, dass man die Siegelfolie luftdicht auf den flachen Rand der Umhüllung aufsiegelt. Für besondere Zwecke kann jedoch das System aus Nährbodenträger, Umhüllung und Siegelfolie zunächst auch ohne Nährboden hergestellt, sterilisiert und vertrieben werden.

Der Nährbodenträger besitzt vorzugsweise eine rechteckige Form, besonders bevorzugt besitzt er die Aussenmasse 100 × 50 mm. Er kann selbstverständlich auch grösser oder kleiner sein.

Die Vertiefung im Nährbodenträger weist vorzugsweise eine Fläche von 25 cm² auf, was ein einfaches Umrechnen auf die Standardangabe Keime pro m²/Fläche erlaubt. Diese Fläche ist in Felder unterteilt, die aufgerastet sind, wodurch der Nährboden besser festgehalten und das Auszählen der Keime erleichtert wird. Vorzugsweise sind die Felder viereckig oder rechteckig und besitzen vorzugsweise eine Fläche von 1 cm², wobei die Trennung der Felder durch Quer- und Längsstege erfolgt. Zur Stabilisierung liegen bei einem rechteckigen Nährbodenträger vorzugsweise die Querstege etwas höher als die Längsstege.

Der die Nährbodenfläche umgebende Wulstrand kann unterschiedlich hoch sein in Abhängigkeit des beabsichtigten Verwendungszweckes. Bei einem hohen Rand und einem Nährbodenniveau unterhalb des Randes lässt sich das System als sogenannte Petrischale verwenden, während bei einem niedrigen Rand, bei dem das Nährbodenniveau über den Rand hinaus gewölbt ist, das System zur Bestimmung von Keimen auf Flächen oder in Flüssigkeiten, d.h. als Abklatschplatte, Keimindikator, Dip-slide etc. verwendet werden kann.

Darüber hinaus lässt sich das System zur mikrobiologischen Sensibilitätsbestimmung (Antibiotikaresistenz) und zur Erregeridentifizierung (bunte Reihe) verwenden.

Bei der Ausführungsform mit hohem Rand beträgt die Randhöhe vorzugsweise 8 mm, während bei der niedrigen Ausführungsform die Randhöhe vorzugsweise 4 mm beträgt. Beide Ausführungsformen lassen sich mit ein und demselben Formwerkzeug fertigen, dessen Randhöhe nur verstellt zu werden braucht. Die Ausführungsform mit niedrigem Rand kann vorzugsweise biegsam sein, was einen besonderen Vorteil bei ihrer Verwendung als Abklatschplatte darstellt.

Ferner kann es bei dieser Ausführungsform zweckmässig sein, den Wulstrand an einer oder mehreren Stellen rinnenartig durch den flachen Rand durchzuführen, um beispielsweise bei der Verwendung in Flussigkeiten ein besseres Ablaufen derselben zu gewährleisten. Wenn der Nährbodenträger mit Nährboden gefüllt ist, befindet sich die Nährbodenebene vorzugsweise etwa 2,5 mm unterhalb des Randes.

In einer besonderen Ausführungsform kann die Vertiefung des Nährbodenträgers nochmals durch eine oder mehrere Wände unterteilt sein, wobei in die einzelnen Abteilungen unterschiedliche Nährmedien eingebracht werden können. An einem Ende besitzt der Nährbodenträger ein Griffstück, damit derselbe gehandhabt werden kann, ohne den Nährboden zu berühren. Zur Stabilisierung kann das Griffstück mit Profilen versehen sein. Geeigneterweise erstreckt sich das Griffstück über die Breite des Nährbodenträgers und besitzt eine Länge von etwa 20 bis 25 mm.

Die Umhüllung setzt sich aus Vertiefung und flachem Rand zusammen. Die Vertiefung, in der der Nährbodenträger Platz findet, ist zum flachen Rand hin stufenförmig auf die Aussenmasse des Nährbodenträgers verbreitert, während der engere Bodenteil so bemessen ist, dass die beiden Seitenwände und eine Endwand den Wulstrand des Nährbodenträgers berühren, und zwar die beiden Seitenwände und die dem Griffstück gegenüberliegende Endwand. Unter Berühren versteht man im vorliegenden, dass die Wände beim Einschieben des Trägers in die Umhüllung aneinander gleiten. Der flache Rand des Trägers, der den Wulstrand umgibt, gleitet auf der Treppenstufe. Am oberen Teil der Stufe befinden sich Festhaltestege, die den Nährbodenträger fixieren. Dadurch lässt sich der Nährbodenträger nach Entfernung der Siegelfolie und nach Beimpfung ohne fremde Vorrichtung fest in die Umhüllung einschieben bzw. einrasten, so dass eine keimfreie Handhabung bis zur Bebrütung gewährleistet ist.

Diese Festhaltestege können im Abstand voneinander um die drei Seiten der Umhüllung herum angebracht sein, sie können jedoch auch kontinuierlich verlaufen.

Die Seite der Umhüllung, an der das Griffstück des Trägers zu liegen kommt, ist abgerundet oder abgeschrägt, so dass der Träger nicht herausrutschen kann. Sie ist ferner mit einer Aussparung derart versehen, dass man mit den Fingern hineingreifen und den Nährbodenträger mühelos greifen und herausziehen kann. Ausserdem dient diese Aussparung dem erforderlichen Gasaustausch.

Für beide Nährbodenträgertypen, d.h. mit hohem und mit niedrigem Wulstrand, kann die gleiche Umhüllung verwendet werden. In jedem Falle müssen die Ausmasse der Vertiefung derart sein, dass zwischen Trägerwulstrand und Umhüllungsboden ein Spalt zum Gasaustausch verbleibt. Die Breite dieses Spalts ist beliebig, beträgt zweckmässigerweise jedoch etwa 1 bis 4 mm.

Auf den flachen Rand der Umhüllung wird die Siegelfolie aufgesiegelt, die das gesamte System luft- und keimdicht abschliesst.

Die Erfindung wird im folgenden anhand der Zeichnungen näher erläutert. Es zeigen:

Fig. 1 eine Draufsicht auf den Nährbodenträger;

Fig. 2 eine Draufsicht auf die Umhüllung, und

Fig. 3 einen Schnitt entlang der Linie A-A der Fig. 1 und Fig. 2.

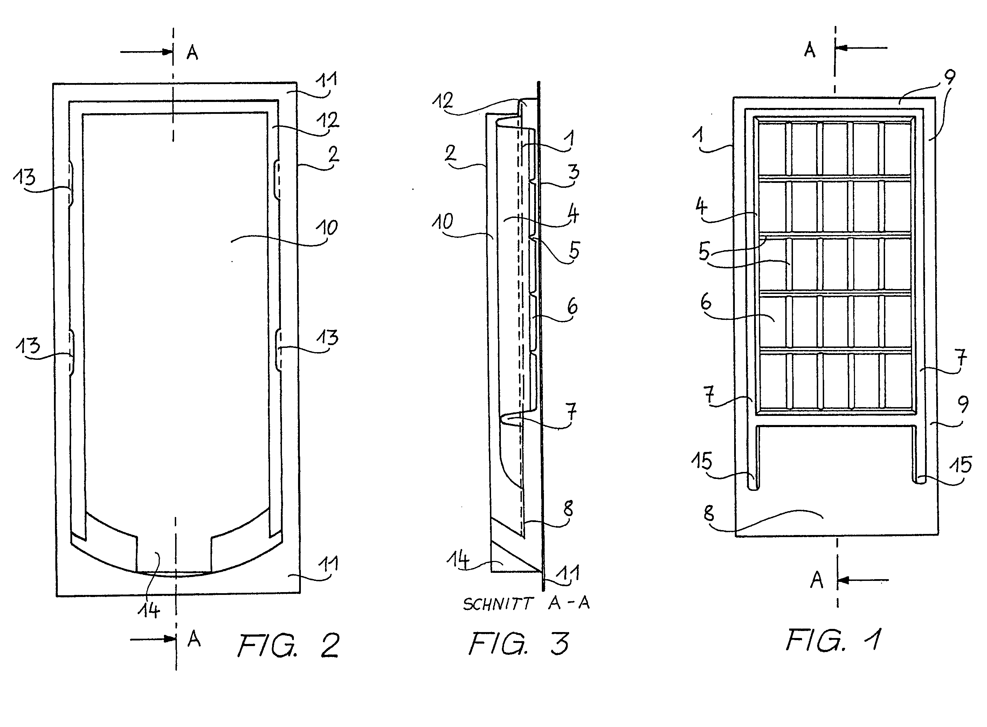

Der in Fig. 1 gezeigte Nährbodenträger 1 weist eine zur Aufnahme des Nährbodens bestimmte rechteckige Vertiefung 4 auf, die durch Stege 5 in rechteckige Felder 6 unterteilt ist. Um die gesamte Vertiefung 4 läuft ein vom Boden Vertiefung nach oben gezogener Wulstrand 7. An einem Ende des Nährbodenträgers ist das Griffstück 8 zu sehen, das mit Stabilisierungsprofilen 15 verstärkt ist. Die Stabilisierungsprofile 15 stellen in dieser Ausführungsform Ausläufer des Wulstrandes 7 dar. Um die gesamte Anordnung läuft ein flacher Rand 9, der mit dem Griffstück 8 in einer Ebene liegt.

Die in Fig. 2 gezeigte Umhüllung 2 weist eine wannenartige Vertiefung 10 auf, die an dem Ende, an dem das Griffstück des Nährbodenträgers zu liegen kommen soll, eine Rundung mit einer Aussparung 14 besitzt. Um die drei geraden Seiten läuft ein Stufenabsatz 12 und um die gesamte Anordnung ein flacher Rand 11 zum Aufbringen einer Siegelfolie, die das gesamte Nährbodenträgersystem hermetisch verschliessen soll. Um die drei geraden Seiten verlaufen an der Innenseite des flachen Randes 11 Festhaltestege 13, die dazu dienen, den Nährbodenträger im eingeschobenen Zustand, auch wenn die Siegelfolie schon entfernt ist, festzuhalten.

Der in Fig. 3 gezeigte Schnitt zeigt einen Aufriss des gesamten erfindungsgemässen Nährbodenträgersystems, worin der Nährbodenträger 1 mit der zur Aufnahme des Nährbodens bestimmten Vertiefung 4 nach unten, d.h. zum Boden der Umhüllung 2 zeigend in letzterer eingeschoben liegt. Das Ganze ist mit Siegelfolie 3, die fest und bündig auf dem flachen Rand 11 der Umhüllung 2 aufliegt, verschlossen. In diesem Aufriss sind der Stufenabsatz 12, der Wulstrand 7, die wannenartige Vertiefung 4 im Nährbodenträger sowie die Stege 5 zu erkennen, die diese Vertiefung nochmals in näpfchenartige Felder 6, die den Nährboden aufnehmen und festhalten, unterteilen. Man erkennt ferner die Lage des Griffstücks 8 in der Vertiefung 10 der Umhüllung 2 und die Aussparung 14, in die man hineingreifen und das Griffstück zu fassen bekommen kann. Man erkennt ferner die Festhaltestege 13, die zusammen mit dem Stufenabsatz 12 eine Art Rille bilden, durch die der flache Rand 9 des Nährbodenträgers 1 und somit der ganze Nährbodenträger festgehalten wird.

## Patentansprüche

1. Nährbodenträgersystem bestehend aus Nährbodenträger (1), Umhüllung (2) und Siegelfolie (3), dadurch gekennzeichnet, dass der Nährbodenträger (1) eine zur Aufnahme des Nährbodens bestimmte Vertiefung (4), die durch Stege (5) in Felder (6) unterteilt ist, einen von der Nährbodenfläche nach oben gezogenen umlaufenden Wulstrand (7), an einem Ende ein Griffstück (8) und einen um beide Seiten und das andere Ende herumlaufenden flachen Rand (9) aufweist und die Umhüllung (2) eine Vertiefung (10) und einen umlaufenden flachen Rand (11) zur Aufnahme der Siegelfolie (3) aufweist, wobei die Vertiefung (10) an zwei Seiten und einem Ende sich zum flachen Rand (11) hin stufenförmig auf die Ausmasse des Nährbodenträgers (1) verbreitert, der engere Teil so bemessen ist, dass die beiden Seitenwände und ein Ende den Wulstrand (7) an zwei Seiten und an dem dem Griffstück (8) gegenüberliegenden Ende berühren, der Stufenabsatz (12) an seinem oberen Rand Festhaltestege (13) aufweist, die den Nährbodenträger (1), dessen flacher Rand (9) auf der Stufe aufliegt, festhalten, und die Vertiefung (10) an der Seite, an der das Griffstück (8) des Nährbodenträgers (1) zu liegen kommt, abgerundet oder abgeschrägt ist und die Rundung oder Schräge mit einer Aussparung (14) zum Greifen und Herausziehen des Nährbodenträgers (1) versehen ist und wobei Nährbodenträger (1) und Umhüllung (2) aus sterilisierbarer Kunststoff-Folie bestehen.

2. Nährbodenträgersystem nach Anspruch 1, dadurch gekennzeichnet, dass Nährbodenträger (1) und Umhüllung (2) eine rechteckige Form aufweisen.

3. Nährbodenträgersystem nach den Ansprüchen 1 oder 2, dadurch gekennzeichnet, dass der Nährbodenträger (1) die Aussenmasse $100 \times 50$ mm aufweist.

4. Nährbodenträgersystem nach einem der Ansprüche 1 bis 3, dadurch gekennzeichnet, dass die den Nährboden aufnehmende Vertiefung (4) eine Fläche von 25 cm² aufweist.

5. Nährbodenträgersystem nach einem der Ansprüche 1 bis 4, dadurch gekennzeichnet, dass die Felder (6) eine Fläche von jeweils 1 cm² aufweisen.

6. Nährbodenträgersystem nach einem der Ansprüche 1 bis 5, dadurch gekennzeichnet, dass die Felder (6) eine rechteckige Form aufweisen.

7. Nährbodenträgersystem nach einem der Ansprüche 2 bis 6, dadurch gekennzeichnet, dass bei den Stegen (5) die Querstege höher liegen als die Längsstege.

8. Nährbodenträgersystem nach einem der Ansprüche 1 bis 7, dadurch gekennzeichnet, dass das Griffstück (8) mit einem Stabilisierungsprofil (15) ausgestattet ist.

9. Nährbodenträgersystem nach einem der Ansprüche 1 bis 8, dadurch gekennzeichnet, dass der Wulstrand (7) eine Höhe von 4 mm aufweist.

10. Nährbodenträgersystem nach einem der Ansprüche 1 bis 8, dadurch gekennzeichnet, dass der Wulstrand (7) eine Höhe von 8 mm aufweist.

11. Nährbodenträgersystem nach einem der Ansprüche 1 bis 10, dadurch gekennzeichnet, dass die Vertiefung (4) einen oder mehrere Unterteilungsstege aufweist.

12. Nährbodenträgersystem nach einem der Ansprüche 1 bis 11, dadurch gekennzeichnet, dass die Vertiefung (4) mit Nährboden gefüllt ist.

13. Nährbodenträgersystem nach Anspruch 12, dadurch gekennzeichnet, dass die Vertiefung (4) bzw. die Felder (6) mit unterschiedlichen Nährbodenarten gefüllt sind.

## Claims

1. A nutrient medium carrier system consisting of a nutrient medium carrier (1), a covering (2), and a sealing foil (3), characterized in that the nutrient medium carrier (1) has a recess (4) designed to receive the nutrient medium, which is subdivided into compartments (6) by means of bars (5), a surrounding beaded edge (7) raised up from the surface of the nutrient medium, a handle (8) on one end, and a flat edge (9) running around both sides and the other end and the covering (2) has a recess (10) and a surrounding flat edge (11) to receive the sealing foil (3), with the recess (10) widening in the form of steps on two sides and one end to the flat edge (11) up to the dimensions of the nutrient medium carrier (1), the narrower part is measured in such a way that both side walls and one end touch the beaded edge (7) on two sides and on the end opposite the handle (8), the steplike offset (12) has locking flanges (13) on its upper edge that hold the nutrient medium carrier (1), the flat edge (9) of which rests on the steplike offset and the recess (10) on the side on which the handle (8) of the nutrient medium carrier (1) comes to lie is rounded off or slanted and the rounded or slanted part is provided with a hollow (14) for gripping and pulling out the nutrient medium carrier (1), the covering (2) consisting of plastic foil that can be sterilized.

2. A nutrient medium carrier system as claimed in claim 1, characterized in that the nutrient medium carrier (1) and the covering (2) have a rectangular shape.

3. A nutrient medium carrier system as claimed in one of claims 1 or 2, characterized in that the nutrient medium carrier (1) has the dimensions 100 × 50 mm.

4. A nutrient medium carrier system as claimed in one of claims 1 to 3, characterized in that the recess (4) that receives the nutrient medium has a surface of 25 cm².

5. A nutrient medium carrier system as claimed in one of claims 1 to 4, characterized in that the compartments (6) have an area of 1 cm² in each case.

6. A nutrient medium carrier system as claimed in one of claims 1 to 5, characterized in that the compartments (6) have a rectangular shape.

7. A nutrient medium carrier system as claimed in one of claims 2 to 6, characterized in that with respect to the bars (5) the crossbars are higher than the longitudinal bars.

8. A nutrient medium carrier system as claimed in one of claims 1 to 7, characterized in that the handle (8) is provided with a stabilizing profile (15).

9. A nutrient medium carrier system as claimed in one of claims 1 to 8, characterized in that the beaded edge (7) has a height of 4 mm.

10. A nutrient medium carrier system as claimed in one of claims 1 to 8, characterized in that the beaded edge (7) has a height of 8 mm.

11. A nutrient medium carrier system as claimed in one of claims 1 to 10, characterized in that the recess (4) has one or several partitioning bars.

12. A nutrient medium carrier system as claimed in one of claims 1 to 11, characterized in that the recess (4) is filled with nutrient medium.

13. A nutrient medium carrier system as claimed in claim 12, characterized in that the recess (4) or the compartments (6) respectively are filled with different types of nutrient medium.

**Revendications**

1. Dispositif porteur de milieu de culture, se composant d'un support (1) de milieu de culture, d'une enveloppe (2) et d'une feuille de scellement (3), caractérisé en ce que le support (1) de milieu de culture présente une cavité (4) destinée à recevoir un milieu de culture et qui est divisée en quartiers (6) par des nervures (5), un rebord à bourrelet (7) d'entourage s'étendant vers le haut à partir de la surface du milieu de culture, à une extrémité une poignée (8) et un bord plat (9) courant tout autour des deux côtés et de l'autre extrémité, en ce que l'enveloppe (2) présente une cavité (10) et un bord plat (11) d'encadrement pour recevoir la feuille de scellement (3), de sorte que la cavité (10) s'étend sur deux côtés et une extrémité jusqu'au bord plat (11) par gradins de la dimension du support (1) de milieu de culture, la partie resserrée étant calculée de façon que les parois latérales et une extrémité viennent toucher le rebord à bourrelet (7) sur deux côtés et sur l'extrémité opposée à la poignée (8), en ce que le gradin (12) présente sur son bord supérieur des nervures de maintien (13) qui retiennent le support (1) de milieu de culture, dont le bord plat repose sur le gradin, et en ce que la cavité (10), du côté sur lequel la poignée (8) du support (1) de milieu de culture vient se placer, est arrondie ou chanfreinée, l'arrondi ou le chanfrein étant doté d'un évidement (14) pour saisir et tirer le support (1) de milieu de culture, le support (1) et l'enveloppe (2) étant constitués d'une feuille stérilisable de matière plastique.

2. Dispositif porteur de milieu de culture selon la revendication 1, caractérisé en ce que le support (1) de milieu de culture et l'enveloppe (2) présentent une forme rectangulaire.

3. Dispositif porteur de milieu de culture selon l'une des revendications 1 ou 2, caractérisé en ce que le support (1) de milieu de culture présente des dimensions de 100 × 50 mm.

4. Dispositif porteur de milieu de culture selon l'une des revendications 1 à 3, caractérisé en ce que la cavité (4) recevant le milieu de culture présente une surface de 25 cm².

5. Dispositif porteur de milieu de culture selon l'une des revendications 1 à 4, caractérisé en ce que les quartiers (6) offrent chacun une surface de 1 cm².

6. Dispositif porteur de milieu de culture selon l'une des revendications 1 à 5, caractérisé en ce que les quartiers (6) présentent une forme rectangulaire.

7. Dispositif porteur de milieu de culture selon l'une des revendications 2 à 6, caractérisé en ce que parmi les nervures (5), les nervures transversales sont plus hautes que les nervures longitudinales.

8. Dispositif porteur de milieu de culture selon l'une des revendications 1 à 7, caractérisé en ce que la poignée (8) est équipée de nervures de rigidité (15).

9. Dispositif porteur de milieu de culture selon l'une des revendications 1 à 8, caractérisé en ce que le rebord à bourrelet (7) présente une hauteur de 4 mm.

10. Dispositif porteur de milieu de culture selon l'une des revendications 1 à 8, caractérisé en ce que le rebord à bourrelet (7) présente une hauteur de 8 mm.

11. Dispositif porteur de milieu de culture selon l'une des revendications 1 à 10, caractérisé en ce que la cavité (4) comporte une ou plusieurs nervures de cloisonnement.

12. Dispositif porteur de milieu de culture selon l'une des revendications 1 à 11, caractérisé en ce que la cavité (4) est remplie de milieu de culture.

13. Dispositif porteur de milieu de culture selon la revendication 12, caractérisé en ce que la cavité (4) ou encore les quartiers (6) sont remplis de diverses sortes de milieu de culture.

FIG. 2

SCHNITT A - A

FIG. 3

FIG. 1

0 045 041